(19) Europäisches Patentamt
European Patent Office
Office européen des brevets

(11) Publication number : 0 459 830 A1

(12) # EUROPEAN PATENT APPLICATION

(21) Application number : 91304963.1

(22) Date of filing : 31.05.91

(51) Int. Cl.[5] : C07D 239/48, C07D 239/46, C07D 239/47, C07D 239/52, A61K 31/505

(30) Priority : 01.06.90 GB 9012311

(43) Date of publication of application : 04.12.91 Bulletin 91/49

(84) Designated Contracting States : AT BE CH DE DK ES FR GB GR IT LI LU NL SE

(71) Applicant : THE WELLCOME FOUNDATION LIMITED
Unicorn House 160 Euston Road
London NW1 2BP (GB)

(72) Inventor : Leach, Michael John
The Wellcome Research Laboratories,
Langley Court
Beckenham, Kent BR3 3BS (GB)
Inventor : Nobbs, Malcolm Stuart
The Wellcome Research Laboratories,
Langley Court
Beckenham, Kent BR3 3BS (GB)

(74) Representative : Stott, Michael John et al
The Wellcome Research Laboratories Group
Patents & Agreements Langley Court
Beckenham Kent BR3 3BS (GB)

(54) **Pharmacologically active CNS compounds.**

(57) The invention relates to compounds of formula (I) and salts thereof

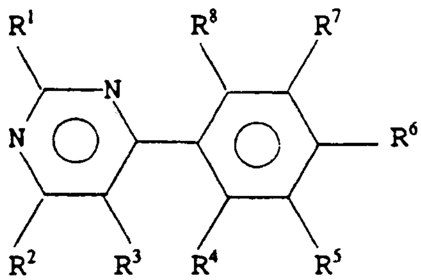

(I)

wherein $R^1$ and $R^2$ are the same or different and are each selected from hydroxy, or a group $NR^9R^{10}$ wherein $R^9$ and $R^{10}$ are each hydrogen, alkyl, aryl, arylalkyl or together with the nitrogen atom to which they are attached form a ring, optionally containing a further heteroatom and optionally further substituted by alkyl, aryl or arylalkyl ;
$R^3$ is selected from hydrogen, haloalkyl, alkyl and halo, $R^4$ to $R^8$ are the same or different, and are each selected from hydrogen, halo, nitro, amino, sulphonamido or alkylsulphonylamino ;
or $R^4$ and $R^5$ or $R^5$ and $R^6$ may together form a carbocyclic ring ;
with the proviso that at least one of $R^4$ to $R^8$ is other than hydrogen.
The compounds may be used for the treatment or prophylaxis of a neurodegenerative or other neurological disorder of the CNS, the aetiology of which includes excessive release of the neurotransmitter glutamate.

EP 0 459 830 A1

The present invention relates to a series of substituted 6-aryl pyrimidines, to methods for their manufacture, to pharmaceutical formulations containing them and to their use in therapy, in particular the treatment of a range of central nervous system (CNS) disorders and disease states.

Glutamate is an excitatory amino acid which functions as a neurotransmitter. However, when its extracellular concentration is sufficiently high, glutamate acts as a powerful neurotoxin, capable of killing neurones in the central nervous system, (Rothman & Olney (1986) Prog.Brain.Res., 63, 69). The neurotoxic effect of glutamate has been implicated in a number of central nervous system disorders and disease states including cerebral ischaemic damage and chronic neurodegenerative disorders, such as Alzheimer's disease, motor system disorders, and Huntington's chorea, (Meldrum Clinical Science (1985) 68 113-122). In addition, glutamate has been implicated in other neurological disorders such as epilepsy, manic depression, depression, schizophrenia, high pressure neurological syndrome, chronic pain, trigeminal neuralgia and migraine.

In EP-A-021121 there is disclosed a group of 3,5-diamino-6-(substituted phenyl)-1,2,4-triazines which are active in the treatment of CNS disorders, for example in the treatment of epilepsy. One compound described in that application, 3,5-diamino-6-(2,3-dichlorophenyl)-1,2,4-triazine (lamotrigine), has been shown to inhibit the release of the excitatory amino acids, glutamate and aspartate, (Leach et al Epilepsia 27, 490-497 1986, A.A. Miller et al New anticonvulsant drugs. Ed. Meldrum and Porter 165-177, 1987).

A novel class of 6-aryl pyrimidine compounds has now been found that are potent inhibitors of extracellular glutamate release.

Accordingly, the present invention provides a compound of formula (I) or a salt thereof

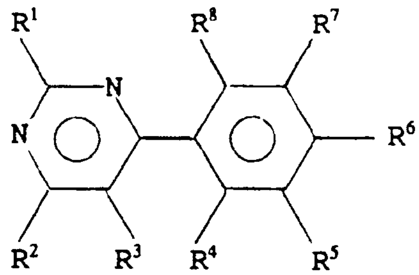

(I)

wherein $R^1$ and $R^2$ are the same or different and are each selected from hydroxy, or a group $NR^9R^{10}$ wherein $R^9$ and $R^{10}$ are each hydrogen, alkyl, aryl, arylalkyl or together with the nitrogen atom to which they are attached form a ring, optionally containing a further heteroatom and optionally further substituted by alkyl, aryl or arylalkyl;
$R^3$ is selected from hydrogen, haloalkyl, alkyl and halo;
$R^4$ to $R^8$ are the same or different, and are each selected from hydrogen, halo-, nitro, amino, sulphonamido or alkylsulphonylamino;
with the proviso that at least one of $R^4$ to $R^8$ is other than hydrogen, and with the further proviso that when $R^1$ and $R^2$ are both amino, $R^3$ is hydrogen or methyl, and $R^6$ is chloro, then one of $R^4$, $R^5$, $R^7$ or $R^8$ is other than hydrogen.

According to a preferred embodiment of the present invention, there is provided a compound of formula (I) or salt thereof, wherein $R^1$ and $R^2$ are the same or different and are each selected from amino, alkylamino, dialkylamino, morpholino, piperazinyl, or N'-alkyl piperazinyl;
$R^3$ is hydrogen, alkyl or trifluoromethyl;
$R^4$ is halo or hydrogen;
$R^5$ is halo or hydrogen;
$R^6$ is halo, nitro, amino or hydrogen;
$R^7$ is halo or hydrogen;
$R^8$ is halo or hydrogen;
provided that when $R^6$ is chloro, and $R^1$ and $R^2$ are both amino and $R^3$ is hydrogen or methyl, then one of $R^4$, $R^5$, $R^7$ and $R^8$ is other than hydrogen.

Preferred alkyl moieties are those with 1 to 6 carbon atoms, more preferably 1 to 4 carbon atoms, and most preferably methyl or ethyl. Preferred halo radicals are chloro.

Three 2,4-diamino-6-aryl pyrimidines are known, namely 2,4-diamino-6-(4-chlorophenyl)pyrimidine; 2,4-diamino-5-methyl-6-(4-chlorophenyl)pyrimidine; and 2,4-diamino-6-(β-naphthyl)pyrimidine (see Russell, J.Chem.Soc. (1954) p2951 and Falco et al., and Brit.J.Pharmacol. (1951) 6, 185). No biological activity has been ascribed to these compounds.

Accordingly the present invention provides a compound of formula (Ia) or a pharmaceutically acceptable salt thereof

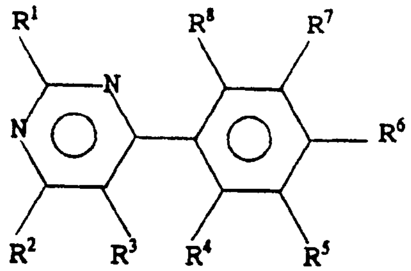

(Ia)

wherein $R^1$ and $R^2$ are the same or different and are each selected from hydroxy, or a group $NR^9R^{10}$ wherein $R^9$ and $R^{10}$ are each hydrogen, alkyl, aryl, arylalkyl or together with the nitrogen atom to which they are attached form a ring, optionally containing a further heteroatom and optionally further substituted by alkyl, aryl or arylalkyl;
$R^3$ is selected from hydrogen, haloalkyl, alkyl and halo;
$R^4$ to $R^8$ are the same or different and are each selected from hydrogen, halo, nitro, amino, sulphonamido or alkylsulphonylamino;
or $R^4$ and $R^5$ or $R^5$ and $R^6$ may together form a carbocyclic ring;
with the proviso that at least one of $R^4$ to $R^8$ is other than hydrogen;
for use in therapy.

According to a preferred embodiment, the invention provides a compound of formula (Ia) or a pharmaceutically acceptable salt thereof wherein $R^1$ and $R^2$ are the same or different and are each selected from amino, alkylamino, dialkylamino, morpholino, piperazinyl, or N'-alkyl piperazinyl;
$R^3$ is hydrogen, alkyl or trifluoromethyl;
$R^4$ is halo or hydrogen;
$R^5$ is halo or hydrogen;
$R^6$ is halo, nitro, amino or hydrogen;
$R^7$ is halo or hydrogen;
$R^8$ is halo or hydrogen;
or $R^5$ and $R^6$ may form a carbocyclic ring;
for use in therapy.

Preferred compounds of formula (I) include:-
2,4-Diamino-6-(2,3-dichlorophenyl)pyrimidine;
2,4-Diamino-6-(2-chlorophenyl)pyrimidine;
2,4-Diamino-6-(2,4-dichlorophenyl)pyrimidine;
2,4-Diamino-5-methyl-6-(4-chlorophenyl)pyrimidine;
2,4-Diamino-6-(α-naphthyl)pyrimidine;
2,4-Diamino-6-(2,3,5-trichlorophenyl)pyrimidine; .
2,4-Diamino-5-methyl-6-(2,3,5-trichlorophenyl)pyrimidine;
2,4-Diamino-5-trifluoromethyl-6-(2,3,5-trichlorophenyl) pyrimidine;
2,4-Diamino-5-nitro-6-(2,3,5-trichlorophenyl)pyrimidine;
2,4-Diamino-5-methyl-6-(4-amino-3,5-dichlorophenyl) pyrimidine;
2,4-Diamino-5-methyl-6-(3,5-dichlorophenyl)pyrimidine;
4-Amino-2-N,N-dimethylamino-6-(2,3,5-trichlorophenyl) pyrimidine;
4-Amino-2-N-morpholino-6-(2,3,5-trichlorophenyl)pyrimidine;
4-Amino-2-(4-methylpiperazin-1-yl)-6-(2,3,5-trichlorophenyl) pyrimidine;
2,4-Diamino-5-methyl-6-(4-amino-2,3,5-trichlorophenyl) pyrimidine;
2,4-Diamino-6-(4-amino-3,5-dichlorophenyl)pyrimidine;
2,4-Diamino-6-(4-amino-2,3,5-trichlorophenyl)pyrimidine.

Compounds of formula (Ia) may be used in the treatment or prophylaxis in a mammal of a neurodegenerative or other neurological disorder of the central nervous system, the aetiology of which includes excessive release of the neurotransmitter glutamate. Such conditions can be either acute or chronic. Acute condition include cerebral ischaemic damage which may arise from a variety of causes including stroke, cardiac arrest, cardiac bypass surgery, neonatal anoxia and hypoglycaemia; and also physical injury or trauma of the spinal cord or brain. Chronic disorders include Alzheimer's disease, Huntington's chorea, Olivopontocerebrellar atrophy and motor system disorders. Other neurological conditions which may be treated with a compound of formula (Ia) include depression, manic depression, schizophrenia, chronic pain, epilepsy, trigeminal neuralgia and migraine.

According to a further aspect, the present invention provides a method for the treatment or prophylaxis in

a mammal, including man, of a neurodegenerative or other neurological disorder of the central nervous system, the aetiology of which includes excessive release of the neurotransmitter glutamate which comprises administering to the said mammal a non-toxiceffective amount of a compound of formula (Ia) or a salt thereof.

In particular, the invention provides a method of treating a mammal predisposed to or having increased extracellular glutamate levels of the CNS comprising the administration to the said mammal of a non-toxic, effective amount of a compound of formula (Ia) or a pharmaceutically acceptable salt thereof.

The invention also provides a compound of formula (Ia) or a salt thereof for use in therapy, in particular the treatment or prophylaxis of a neurodegenerative or other neurological disorder of the central nervous system, the aetiology of which includes excessive release of the neurotransmitter glutamate. The invention further provides the use of a compound of formula (Ia) or a salt thereof for the manufacture of a medicament for treatment or prophylaxis of a neurodegenerative or other neurological disorder of the central nervous system, the aetiology of which includes excessive release of the neurotransmitter glutamate.

The present invention includes compounds of formula (I) and (Ia) in the form of salts, in particular acid addition salts. Suitable salts include those formed with both organic or inorganic acids. Such acid addition salts will normally be pharmaceutically acceptable although salts of non-pharmaceutically acceptable acids may be of utility in the preparation and purification of the compound in question. Thus, preferred salts include those formed from hydrochloric, hydrobromic, sulphuric, citric, tartaric, phosphoric, lactic, pyruvic, acetic, succinic, oxalic, fumaric, maleic, oxaloacetic, methanesulphonic, ethanesulphonic, p-toluenesulphonic, benzenesulphonic and isethionic acids. Salts of the compounds of formulae (I) and (Ia) can be made by reacting the appropriate compound in the form of the free base with the appropriate acid.

While it is possible for the compounds of formula (Ia) to be administered as the raw chemical, it is preferable to present them as a pharmaceutical formulation. The formulations of the present invention comprise a compound of formula (Ia), as above defined, or a pharmaceutically acceptable salt thereof together with one or more pharmaceutically acceptable carriers therefor and optionally other therapeutic ingredients. The carrier(s) must be 'acceptable' in the sense of being compatible with the other ingredients of the formulation and not deleterious to the recipient thereof.

The formulations include those suitable for oral, parenteral (including subcutaneous, intradermal, intramuscular and intravenous), rectal and topical (including dermal, buccal and sublingual) administration although the most suitable route may depend upon for example the condition and disorder of the recipient. The formulations may conveniently be presented in unit dosage form and may be prepared by any of the methods well known in the art of pharmacy. All methods include the step of bringing into association a compound of formula (Ia) or a pharmaceutically acceptable salt thereof ("active ingredient") with the carrier which constitutes one or more accessory ingredients. In general the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example, water-for-injection, immediately prior to use. Extemporaneous injection solutions and suspensions may be prepared from sterile powders, granules and tablets of the kind previously described.

Formulations for rectal administration may be presented as a suppository with the usual carriers such as cocoa butter or polyethylene glycol.

Formulations for topical administration in the mouth, for example buccally or sublingually, include lozenges comprising the active ingredient in a flavoured basis such as sucrose and acacia or tragacanth, and pastilles

comprising the active ingredient in a basis such as gelatin and glycerin or sucrose and acacia.

Preferred unit dosage formulations are those containing an effective dose, an hereinbelow recited, or an appropriate fraction thereof, of the active ingredient.

It should be understood that in addition to the ingredients particularly mentioned above, the formulations of this invention may include other agents conventional in the art having regard to the type of formulation in question, for example those suitable for oral administration may include flavouring agents.

Tablets or other forms of presentation provided in discrete units may conveniently contain an amount of compound of formula (Ia) which is effective at such dosage or as a multiple of the same, for instance, units containing 5mg to 500mg, usually around 10mg to 250mg.

The compounds of the formula (Ia) are preferably used to treat CNS disorders or diseases by oral administration or injection (intraparenteral or subcutaneous). The precise amount of compound administered to a patient will be the responsibility of the attendant physician. However the dose employed will depend on a number of factors, including the age and sex of the patient, the precise disorder being treated, and its severity. Thus for example when treating a patient with epilepsy the dose range is likely to be significantly lower than when treating a patient after stroke to alleviate cerebral ischaemic damage. Moreover, the route of administration is likely to vary depending on the condition and its severity.

Nonetheless, for treating a CNS disorder such as to alleviate cerebral ischaemic damage as herein described compounds of formula (Ia) may typically be administered either orally or by injection at a dose of from 0.1 to 30 mg/kg per day. The dose range for adult humans is generally in the range from 8 to 2,400 mg/day and preferably in the order of 35 to 1050 mg/day.

Compounds of the present invention may be made by any methods known in the art to make analogous compounds (see, for example, JACS 73, (1951) p3763).

The present invention provides a method of manufacturing a compound of formula (Ia) which comprises reacting a compound of formula (II)

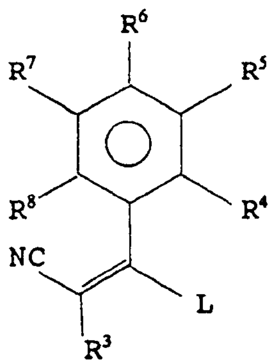

(II)

wherein $R^4$, $R^5$, $R^6$, $R^7$ and $R^8$ are as defined above and L is a leaving group with a compound of formula (III)

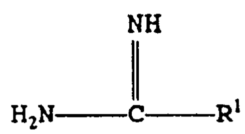

(III)

or a salt thereof, wherein $R^1$ is as defined above and isolating the compound of formula (Ia) as the free base or a salt thereof and optionally converting one compound of formula (Ia) into another compound of formula (Ia) and optionally converting the compound of formula (Ia) in the form of the free base into a salt thereof, or optionally converting one salt into another salt thereof.

Certain compounds of formula (III) may of course exist in corresponding tautomeric form. For example, when $R^1$ is hydroxy as the corresponding urea.

Examples of suitable leaving groups L include alkoxy, particularly $C_{1-4}$alkoxy, halo, alkylthio, particularly $C_{1-4}$alkylthio or substituted amino, eg. anilino, morpholino, $C_{1-4}$alkylamino or benzylamino. Preferably in formula (III), $R^1$ is a group $NR^9R^{10}$ as defined above. Advantageously $R^1$ is amino, alkylamino, dialkylamino, morpholino, piperazinyl or N-methylpiperazinyl.

Preferably the reaction of the compounds of formula (II) and (III) is carried out in a non-aqueous solvent, for example an alkanol, eg. ethanol, at elevated temperatures (eg. from 80 to 150°C). A base, preferably an

alkanoxide, eg. sodium ethoxide is generally added where the compound of formula (III) is used as a salt.

Compound of formula (II) may be made by methods known in the art (see, for example, JACS, 1951, 73, 3763) for example by reaction of the β-ketonitrile with diazomethane.

Compounds of formula (III) when $R^1$ is piperazinyl or alkyl piperazinyl can be made by standard methods for example by reaction of a known compound of formula (III) where $R^1$ is alkylthio with the appropriate amine, eg. N-methylpiperazine. This reaction is preferably carried out at room temperature in a suitable solvent such as water.

The invention is illustrated by the following examples.

Example 1

2,4-Diamino-6-(2,3-dichlorophenyl)pyrimidine

(i) Methyl 2,3-Dichlorobenzoate

2,3-Dichlorobenzoic acid (50g:Aldrich) was refluxed with thionyl chloride (250m1) for 1 hour. The solution was filtered and evaporated to dryness under reduced pressure. The resulting oil was added dropwise to dry methanol (200ml) with stirring at room temperature. After 1 hour the resulting solution was evaporated to dryness under reduced pressure and the residue taken up in ether. The organic phase was washed twice with water, charcoaled, filtered and dried over magnesium sulphate. Yield, 34gms, Mp. 36-38°C.

(ii) 2,3-dichlorobenzoylacetonitrile

Sodium metal (2.3g) was dissolved in dry methanol (100ml) and the solution they evaporated to dryness under reduced pressure. To this residue were added dry acetonitrile (26ml) and methyl 2,3-dichlorobenzoate (20.5g) and the mixture stirred and refluxed under nitrogen overnight. Total was poured into water (100ml) and extracted with ether. The aqueous phase was then carefully acidified to pH6 with 2N hydrochloric acid, and extracted with chloroform. The organic phase was then washed with water, dried over magnesium sulphate, filtered and evaporated to dryness. The resulting oil was purified by column chromatography on silica, eluting with chloroform. Yield 1.75g.

(iii) 3-(2,3-Dichlorophenyl)-3-metoxy-acrylonitrile

2,3-Dichlorobenzoylacetonitri1e (2.8g) was dissolved in ether and this solution added to an excess of diazomethane in ether solution, with stirring and ice-bath cooling. The solution was gradually allowed to warm to room temperature and allowed to stand overnight. The solution was evaporated to dryness under reduced pressure and the crude product purified by column chromatography on silica, eluting with chloroform. The resulting solid was recrystallised from methanol. Yield 1.47g, Mp. 93-95°C.

(iv) 2,4-Diamino-6-(2,3-dichlorophenylpyrimidine

The enol ether (0.7g) was added to a suspension of guanidine (from guanidine hydrochloride (0.67g) and sodium metal (0.16g)) in dry ethanol (40ml) and total heated to 130°C in a sealed vessel for 18 hours. The reaction mixture was evaporated to low volume, taken up in chloroform and washed with water. The organic phase was dried over magnesium sulphate, filtered and evaporated to dryness. The crude product was purified by column chromatography on silica, eluting with chloroform, and 2% methanol in chloroform. Yield 0.15g.

This product was re-chromatographed on silica, eluting with chloroform, and 2% and 5% methanol in chloroform. Yield 0.09g, Mp 180-2°C.

NMR: Solvent = $CDCl_3$

Assignment: 7.50 (d,1H), 7.38(d,1H), 7.25(t,1H), 6.05(s,1H), 4.60-4.90(br.d,4H).

Example 2

2,4-Diamino-6-(2-chlorophenyl)pyrimidine

(i) 3-(chlorophenyl)-3-metoxy-acrylonitrile

2-Chlorobenzoy1acetonitri1e (5g: Lancaster Synthesis) was dissolved in the minimum of dichloromethane

and added to an excess of diazomethane in ether solution, with stirring and ice-bath cooling. The solution was gradually allowed to warm to room temperature and left to stand overnight. The solution was evaporated to dryness under reduced pressure and the crude product purified by column chromatography on silica, eluting with dichloromethane. Yield 4.85g, Mp. 42-44°C.

(ii) 2,4-Diamino-6-(2-chlorophenyl)pyrimidine

The enol ether (4.75g) was added to a suspension of guanidine (from guanidine hydrochloride (4.68g) and sodium metal (1.13g)) in dry ethanol (125ml) and the total heated to 150°C in a sealed vessel for 18 hours. The reaction mixture was evaporated to low volume, taken up in chloroform and washed with water. The organic phase was dried over magnesium sulphate, filtered and evaporated to dryness. The crude product was purified by column chromatography on silica, eluting with chloroform, and 2% methanol in chloroform. Yield 0.5g. This product was re-chromatographed on silica, eluting with chloroform, and 2% methanol in chloroform. Mp. 165-7°C.
NMR: Solvent = $CDCl_3$.
Assignment 6.85-7.15 (m,4H), 5.70(s,1H), 5.40(br.s,2H), 4.87(br.s,2H).

Example 3

2,4-Diamino-6-(2,4-Dichlorophenyl)pyrimidine

(i) Methyl 2,4-Dichlorobenzoate

2,4-Dichlorobenzoic acid (50g: Aldrich) was refluxed with thionyl chloride (250ml) for 1 hour. The solution was filtered and evaporated to drynesn under reduced pressure. The resulting oil was added dropwise to dry methanol (200ml) with stirring at room temperature. After 1 hour the resulting solution was evaporated to dryness under reduced pressure and the residue taken up in ether. The organic phase was washed twice with water, charcoaled filtered and dried over magnesium sulphate. Yield 40g of a yellow oil.

(ii) 2 4-Dichlorobenzoylacetonitrile

Sodium metal (2.3g) was dissolved in dry methanol (100ml) and the solution then evaporated to dryness under reduced pressure. To this residue were added dry acetonitrile (26ml) and methyl 2,4-dichlorobenzoate (20.5g) and the mixture stirred and refluxed under nitrogen overnight. Total was poured into water (100ml) and extracted with ether. The aqueous phase was then carefully acidified to pH5-6 with 2N hydrochloric acid, and extracted with chloroform. The organic phase was then washed with water, dried over magnesium sulphate, filtered and evaporated to dryness. The resulting oil was purified by column chromatography on silica eluting with chloroform. Yield 2.42g of a pale lemon solid.

(iii) 3-(2,4-Dichloropheny)-3-methoxy-acrylonitrile

2,4-Dichlorobenzoylacetonitrile (5.5g) was dissolved/suspended in ether (100ml) and stirred in an ice bath whilst a solution of diazomethane (c.1.25g) in ether (150ml) was added dropwise. The solution was gradually allowed to warm to room temperature and allowed to stand overnight. The solution was evaporated to dryness under reduced pressure and the crude product purified by column chromatography on silica eluting with dichloromethane. Yield 4.8g.

(iv) 2,4-Diamino-6-(2,4-Dichlorophenyl)pyrimidine

The enol ether (2.8g) was added to a suspension of guanidine [from guanidine hydrochioride (2.68g) and sodium metal (0.64g)] in dry ethanol (100ml) and total heated to 160°C in a sealed vessel for 18 hours. Reaction mixture was evaporated to low volume, taken up in chloroform and washed with water. Organic phase was dried over magnesium sulphate filtered and evaporated to dryness. The crude product was twice purified by column chromatography on silica, eluting with chloroform, and 2% methanol in chloroform. Yield 0.21g. mp. = 170-4°C.
NMR: Solvent = $CDCl_3$
Assignment 7.50 (d,1H), 7.47 (s,1H), 7.32 (dd,1H), 6.12 (s,1H), 4.60-5.00 (br.d, 4H).

Example 4

2,4-Diamino-6-(1-naphthyl)pyrimidine

(i) Methyl 1-Naphthoate

1-Naphthoic acid (50g: Aldrich) was refluxed with thionyl chloride (250ml) for 1 hour. The solution was treated with charcoal, filtered and evaporated to dryness under reduced pressure. The resulting oil was added dropwise to dry methanol (200ml) with stirring at room temperature. After 1 hour the resulting solution was evaporated to dryness under reduced pressure and the residue taken up in ether. The organic phase was washed twice with water, charcoaled, filtered and dried over magnesium sulphate. Yield 38g. of a yellow oil.

(ii) 1-Naphthoylacetonitrile

Sodium metal (1.15g) was dissolved in dry methanol (30ml) and solution then evaporated to dryness under reduced pressure. To this residue were added dry acetonitrile (13.5ml) and methyl 1-naphthoate (9.3g) and the mixture stirred and refluxed under nitrogen overnight. Total was poured into water (50ml) and extracted with ether. The aqueous phase was then carefully acidified to pH6-7 with 2N hydrochloric acid and the resulting solid filtered off and washed with water. The crude product was purified by column chromatography on silica, eluting with chloroform. Yield 0.93g. mp. = 95-96°C.

(iii) 3-(1-Naphthy1)-3-methoxy acrylonitrile

1-Naphthoylacetonitrile (6.9g) was dissolved in dichloromethane (100ml) and this solution added to an excess of diazomethane in ether solution with stirring and ice-bath cooling. The solution was gradually allowed to warm to room temperature and allowed to stand overnight. The solution was evaporated to dryness under reduced pressure and the crude product purified by column chromatography on silica, eluting with chlorofom. Yield 6.6g.

(iv) 2,4-Diamino-6-1naphthy)pyrimidine

The enol ether (3.8g) was added to a suspension of guanidine [from guanidine hydrochloride (3.44g) and sodium metal (0.83g)] in dry ethanol (150ml) and total heated to 150°C in a sealed vessel for 18 hours. Reaction mixture was evaporated to low volume, taken up in chloroform and washed with water. Organic phase was dried over magnesium sulphate, filtered and evaporated to dryness. The crude product was purified by column chromatography on silica, eluting with chloroform, and 5% methanol in chloroform. Yield 1.1g. mp. = 93-95°C.
NMR: Solvent = $CDCl_3$
Assignment 8.15 (m,1H), 7.85 (m,2H), 7.50 (m,4H), 6.08 (s,1H), 5.00 (br.s,2H), 4.80 (br.s, 2H).

Pharmacological Activity

Inhibition of Glutamate release and Inhibition of Rat Liver DHFR

Compounds of formula (Ia) were tested for their effect on veratrine-evoked release of glutamate from rat brain slices according to the protocol described in Epilepsia 27(5): 490-497, 1986. The protocol for testing for inhibition of DHFR activity was a modification of that set out in Biochemical Pharmacology Vol.20 pp 561-574, 1971.

The results are given in Table 1, the $IC_{50}$ being the concentration of compound to cause 50% of DHFR enzyme activity.

TABLE 1

| Compound of Example No. | $IC_{50}$ ($\mu M$) Glutamate Release (P95 limits) | $IC_{50}$ ($\mu M$) Rat Liver DHFR (P95 limits) |
|---|---|---|
| 1 | 10 | <100 |
| 2 | 10 | >100 |
| 3 | >10 | <100 |
| * | >10 | >55 |
| 4 | >10 | 100 |

* 2,4-Diamino-5-methyl-6-(4-chlorophenyl)pyrimidine made by the method of Russell, J.Chem.Soc. (1954) 2951.

Pharmaceutical Formulation Example

A: Tablets

| | |
|---|---|
| Compound of Example 1 | 150 mg ) |
| Lactose | 200 mg ) |
| Maize Starch | 50 mg ) |
| Polyvinylpyrrolidone | 4 mg ) |
| Magnesium Stearate | 4 mg ) |

) = contents per tablet.

The drug was mixed with the lactose and starch and granulated with a solution of the polyvinylpyrrolidone in water. The resultant granules were dried, mixed with magnesium stearate and compressed to give tablets.

B: Injections

Injections I

The salt of the compound of Formula (Ia) was dissolved in sterile water for injection.

Intravenous Injection Formulation II

| | |
|---|---|
| Active ingredient | 0.20g |
| Sterile, pyrogen-free phosphate buffer (pH9.0) to | 10ml |

The compound of Example I as a salt is dissolved in most of the phosphate buffer at 35-40°C, then made up to volume and filtered through a sterile micropore filter into sterile 10ml glass vials (Type 1) which are sealed

with sterile closures and overseals.

In the following Examples, the active compound may be any compound of formula (Ia) or pharmaceutically acceptable salt thereof.

C: Capsule Formulations

Capsule Formulation I

Formulation A may be prepared by admixing the ingredients and filling two-part hard gelatin capsules with the resulting mixture.

| | | mg/capsule |
|---|---|---|
| (a) | Active ingredient | 250 |
| (b) | Lactose B.P. | 143 |
| (c) | Sodium Starch Glycollate | 25 |
| (d) | Magnesium Stearate | 2 |
| | | 420 |

Capsule Formulation II

| | | mg/capsule |
|---|---|---|
| (a) | Active Ingredient | 250 |
| (b) | Macrogel 4000 BP | 350 |
| | | 600 |

Capsules may be prepared by melting the Macrogel 4000 BP, dispersing the active ingredient in the melt, and filling two-part hard gelatin capsules therewith.

Capsule Formulation III (Controlled release capsule)

| | | mg/capsule |
|---|---|---|
| (a) | Active Ingredient | 250 |
| (b) | Microcrystalline Cellulose | 125 |
| (c) | Lactose BP | 125 |
| (d) | Ethyl Cellulose | 13 |
| | | 513 |

The controlled-release capsule formulation may be prepared by extruding mixed ingredients (a) to (c) using an extruder, then spheronising and drying the extrudate. The dried pellets are coated with ethyl cellulose (d) as a controlled-release membrane and filled into two-part hard gelatin capsules.

D: Syrup formulation

| | |
|---|---|
| Active ingredient | 0.2500 g |
| Sorbitol Solution | 1.5000 g |
| Glycerol | 1.0000 g |
| Sodium Benzoate | 0.0050 g |
| Flavour | 0.0125 ml |
| Purified Water q.s. to | 5.0 ml |

The sodium benzoate is dissolved in a portion of the purified water and the sorbitol solution added. The

active ingredient is added and dissolved. The resulting solution is mixed with the glycerol and then made up to the required volume with the purified water.

E: Suppository formulation

| | mg/suppository |
|---|---|
| Active ingredient (63μl)* | 250 |
| Hard Fat, BP | |
| (Witepsol H15 - Dynamit Nobel) | 1770 |
| | 2020 |

* The active ingredient is used as a powder wherein at least 90% of the particles are of 63μm diameter or less.

One fifth of the Witepsol H15 is melted in a steam-jacketed pan at 45°C maximum. The active ingredient is sifted through a 200μm sieve and added to the molten base with mixing, using a Silverson fitted with a cutting head, until a smooth dispersion is achieved. Maintaining the mixture at 45°C, the remaining Witepsol H15 is added to the suspension which is stirred to ensure a homogenous mix. The entire suspension is then passed through a 250μm stainless steel screen and, with continuous stirring, allowed to cool to 440°C. At a temperature of 38-40°C, 2.02g aliquots of the mixture are filled into suitable plastic moulds and the suppositories allowed to cool to room temperature.

## Claims

1. A compound of formula (I) or a salt thereof

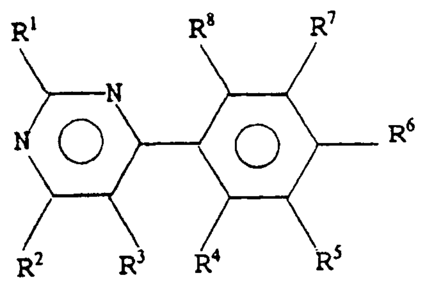

(I)

wherein $R^1$ and $R^2$ are the same or different and are each selected from hydroxy, or a group $NR^9R^{10}$ wherein $R^9$ and $R^{10}$ are each hydrogen, alkyl, aryl, arylalkyl or together with the nitrogen atom to which they are attached form a ring, optionally containing a further heteroatom and optionally further substituted by alkyl, aryl or arylalkyl;
$R^3$ is selected from hydrogen, haloalkyl, alkyl and halo,
$R^4$ to $R^8$ are the same or different, and are each selected from hydrogen, halo, nitro, amino, sulphonamido or alkylsulphonylamino;
with the proviso that at least one of $R^4$ to $R^8$ is other than hydrogen, and with the further proviso that when $R^1$ and $R^2$ are both amino, $R^3$ is hydrogen or methyl, and $R^6$ is chloro, then one of $R^4$, $R^5$, $R^7$ or $R^8$ is other than hydrogen.

2. A compound as claimed in Claim 1 or a salt thereof wherein $R^1$ and $R^2$ are the same or different and are each selected from amino, alkylamino, dialkylamino, morpholino, piperazinyl, or N'-alkyl piperazinyl;
$R^3$ is hydrogen, alkyl or trifluoromethyl;
$R^4$ is halo or hydrogen;

$R^5$ is halo or hydrogen;
$R^6$ is halo, nitro, amino or hydrogen;
$R^7$ is halo or hydrogen;
$R^8$ is halo or hydrogen;
provided that when $R^6$ is chloro, and $R^1$ and $R^2$ are both amino and $R^3$ is hydrogen or methyl, then one of $R^4$, $R^5$, $R^7$ and $R^8$ is other than hydrogen.

**3.** 2,4-Diamino-6-(2,3-dichlorophenyl)pyrimidine;
2,4-Diamino-6-(2-chlorophenyl)pyrimidine;
2,4-Diamino-6-(2,4-dichlorophenyl)pyrimidine;
2,4-Diamino-5-methyl-6-(4-chlorophenyl)pyrimidine;
2,4-Diamino-6-(α-naphthyl)pyrimidine;
2,4-Diamino-6-(2,3,5-trichlorophenyl)pyrimidine;
2,4-Diamino-5-methyl-6-(2,3,5-trichlorophenyl)pyrimidine;
2,4-Diamino-5-trifluoromethyl-6-(2,3,5-trichlorophenyl) pyrimidine;
2,4-Diamino-5-nitro-6-(2,3,5-trichlorophenyl)pyrimidine;
2,4-Diamino-5-methyl-6-(4-amino-3,5-dichlorophenyl)pyrimidine;
2,4-Diamino-5-methyl-6-(3,5-dichlorophenyl)pyrimidine;
4-Amino-2-N,N-dimethylamino-6-(2,3,5-trichlorophenyl) pyrimidine;
4-Amino-2-N-morpholino-6-(2,3,5-trichlorophenyl)pyrimidine;
4-Amino-2-(4-methylpiperazin-1-yl)-6-(2,3,5-trichlorophenyl) pyrimidine;
2,4-Diamino-5-methyl-6-(4-amino-2,3,5-trichlorophenyl) pyrimidine;
2,4-Diamino-6-(4-amino-3,5-dichlorophenyl)pyrimidine;
2,4-Diamino-6-(4-amino-2,3,5-trichlorophenyl)pyrimidine;
or a salt thereof.

**4.** A compound of formula (Ia) or a pharmaceutically acceptable salt thereof

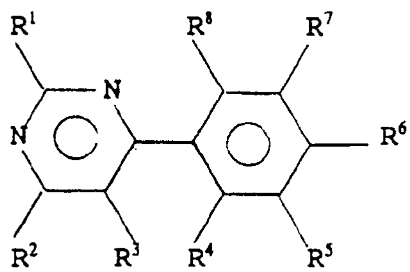

(Ia)

wherein $R^1$ and $R^2$ are the same or different and are each selected from hydroxy, or a group $NR^9R^{10}$ wherein $R^9$ and $R^{10}$ are each hydrogen, alkyl, aryl, arylalkyl or together with the nitrogen atom to which they are attached form a ring, optionally containing a further heteroatom and optionally further substituted by alkyl, aryl or arylalkyl;
$R^3$ is selected from hydrogen, haloalkyl, alkyl and halo, $R^4$ to $R^8$ are the same or different, and are each selected from hydrogen, halo, nitro, amino, sulphonamido or alkylsulphonylamino;
or $R^4$ and $R^5$ or $R^5$ and $R^6$ may together form a carbocyclic ring;
with the proviso that at least one of $R^4$ to $R^8$ is other than hydrogen;
for use in therapy.

**5.** A compound as claimed in Claim 4 or a pharmaceutically acceptable salt thereof wherein $R^1$ and $R^2$ are the same or different and are each selected from amino, alkylamino, dialkylamino, morpholino, piperazinyl, or N'-alkyl piperazinyl;
$R^3$ is hydrogen, alkyl or trifluoromethyl;
$R^4$ is halo or hydrogen;
$R^5$ is halo or hydrogen;
$R^6$ is halo, nitro, amino or hydrogen;
$R^7$ is halo or hydrogen;
$R^8$ is halo or hydrogen;
or $R^4$ and $R^5$ may together form a carbocyclic ring;

for use in therapy.

6. A compound as claimed in Claim 4 selected from
2,4-Diamino-6-(2,3-dichlorophenyl)pyrimidine;
2,4-Diamino-6-(2-chlorophenyl)pyrimidine;
2,4-Diamino-6-(2,3-dichlorophenyl)pyrimidine;
2,4-Diamino-5-methyl-6-(4-chlorophenyl)pyrimidine;
2,4-Diamino-6-(α-naphthyl)pyrimidine;
2,4-Diamino-6-(2,3,5-trichlorophenyl)pyrimidine;
2,4-Diamino-5-methyl-6-(2,3,5-trichlorophenyl)pyrimidine;
2,4-Diamino-5-trifluoromethyl-6-(2,3,5-trichlorophenyl) pyrimidine;
2,4-Diamino-5-nitro-6-(2,3,5-trichlorophenyl)pyrimidine;
2,4-Diamino-5-methyl-6-(4-amino-3,5-dichlorophenyl) pyrimidine;
2,4-Diamino-5-methyl-6-(3,5-dichlorophenyl)pyrimidine;
4-Amino-2-N,N-dimethylamino-6-(2,3,5-trichlorophenyl) pyrimidine;
4-Amino-2-N-morpholino-6-(2,3,5-trichlorophenyl)pyrimidine;
4-Amino-2-(4-methylpiperazin-1-yl)-6-(2,3,5-trichlorophenyl) pyrimidine;
2,4-Diamino-5-methyl-6-(4-amino-2,3,5-trichlorophenyl) pyrimidine;
2,4-Diamino-6-(4-amino-3,5-dichlorophenyl)pyrimidine;
2,4-Diamino-6-(4-amino-2,3,5-trichlorophenyl)pyrimidine;
or a pharmaceutically acceptable salt thereof, for use in therapy.

7. A compound as claimed in any of Claims 4 to 6 for use in the therapy or prophylaxis or a neuro-degenerative or other neurological disorder of the central nervous system, the aetiology of which includes excessive release of the neurotransmitter glutamate.

8. A pharmaceutical composition which comprises a compound of formula (Ia) as defined in any of Claims 4 to 6 or a pharmaceutically acceptable salt thereof together with at least one pharmaceutically acceptable carrier.

9. A process for the preparation of a compound of formula (Ia) as defined in any of Claims 4 to 6 which comprises reacting a compound of formula (II)

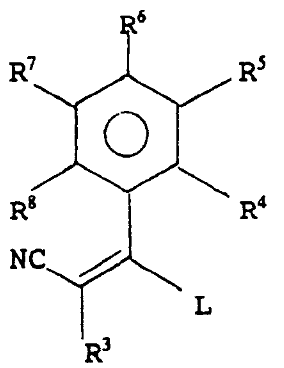

(II)

wherein $R^4$, $R^5$, $R^6$, $R^7$ and $R^8$ are as defined in Claims 4 to 6 and L is a leaving group, with a compound of formula (III)

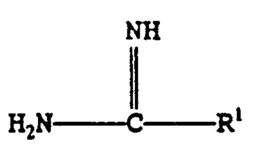

(III)

or a salt thereof, wherein $R^1$ is as defined in any of Claims 4 to 6;
and isolating the compound of formula (Ia) as the free base or a salt thereof;
and optionally converting one compound of formula (Ia) into another compound of formula (Ia); and optionally converting the compound of formula (Ia) in the form of the free base into a salt thereof, or

optionally converting one salt into another salt.

**Claims for the following Contracting States: ES,GR**

1. A process for the preparation of a compound of formula (Ia) or a salt thereof

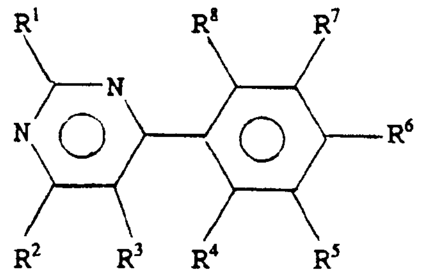

(Ia)

wherein $R^1$ and $R^2$ are the same or different and are each selected from hydroxy, or a group $NR^9R^{10}$ wherein $R^9$ and $R^{10}$ are each hydrogen, alkyl, aryl, arylalkyl or together with the nitrogen atom to which they are attached form a ring, optionally containing a further heteroatom and optionally further substituted by alkyl, aryl or arylalkyl;
$R^3$ is selected from hydrogen, haloalkyl, alkyl and halo,
$R^4$ to $R^8$ are the same or different, and are each selected from hydrogen, halo, nitro, amino, sulphonamido or alkylsulphonylamino;
or $R^4$ and $R^5$ or $R^5$ and $R^6$ may together form a carbocyclic ring;
with the proviso that at least one of $R^4$ to $R^8$ is other than hydrogen;
which comprises reacting a compound of formula (II)

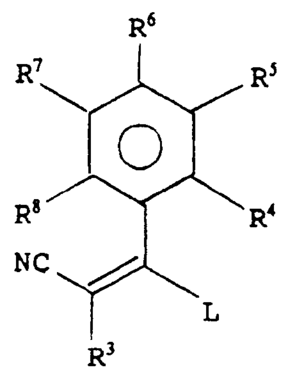

(II)

wherein $R^4$, $R^5$, $R^6$, $R_7$ and $R^8$ are as defined above and L is a leaving group, with a compound of formula (III)

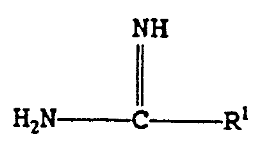

(III)

or a salt thereof, wherein $R^1$ is as defined above;
and isolating the compound of formula (Ia) as the free base or a salt thereof;
and optionally converting one compound of formula (Ia) into another compound of formula (Ia); and optionally converting the compound of formula (Ia) in the form of the free base into a salt thereof, or optionally converting one salt into another salt.

2. A process as claimed in Claim 1 for the production of a compound wherein:
$R^1$ and $R^2$ are the same or different and are each selected from amino, alkylamino, dialkylamino, morpholino, piperazinyl, or N'-alkyl piperazinyl;
$R^3$ is hydrogen, alkyl or trifluoromethyl;

$R^4$ is halo or hydrogen;
$R^5$ is halo or hydrogen;
$R^6$ is halo, nitro, amino or hydrogen;
$R^7$ is halo or hydrogen;
$R^8$ is halo or hydrogen;
or $R^4$ and $R^5$ may together form a carbocyclic ring;
provided that when $R^6$ is chloro, and $R^1$ and $R^2$ are both amino and $R^3$ is hydrogen, then one of $R^4$, $R^5$, $R^7$ and $R^8$ is other than hydrogen.

3. A process as claimed in Claim 1 for the production of a compound selected from:
2,4-Diamino-6-(2,3-dichlorophenyl)pyrimidine;
2,4-Diamino-6-(2-chlorophenyl)pyrimidine;
2,4-Diamino-6-(2,4-dichloropheny1)pyrimidine;
2,4-Diamino-5-methyl-6-(4-chloropheny1)pyrimidine;
2,4-Diamino-6-(α-naphthyl)pyrimidine;
2,4-Diamino-6-(2,3,5-trichlorophenyl)pyrimidine;
2,4-Diamino-5-methyl-6-(2,3,5-trichlorophenyl)pyrimidine;
2,4-Diamino-5-trifluoromethyl-6-(2,3,5-trichlorophenyl) pyrimidine;
2,4-Diamino-5-nitro-6-(2,3,5-trichlorophenyl)pyrimidine;
2,4-Diamino-5-methyl-6-(4-amino-3,5-dichlorophenyl) pyrimidine;
2,4-Diamino-5-methyl-6-(3,5-dichlorophenyl)pyrimidine;
4-Amino-2-N,N-dimethylamino-6-(2,3,5-trichlorophenyl) pyrimidine;
4-Amino-2-N-morpholino-6-(2,3,5-trichlorophenyl)pyrimidine;
4-Amino-2-(4-methylpiperazin-1-yl)-6-(2,3,5-trichlorophenyl) pyrimidine;
2,4-Diamino-5-methyl-6-(4-amino-2,3,5-trichlorophenyl) pyrimidine;
2,4-Diamino-6-(4-amino-3,5-dichlorophenyl)pyrimidine;
2,4-Diamino-6-(4-amino-2,3,5-trichlorophenyl)pyrimidine;
or a salt thereof.

European Patent Office

# EUROPEAN SEARCH REPORT

Application Number

EP 91304963.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE - A1 - 3 008 693 (UPJOHN) * Claims 1,34,36 * -- | 1,7,8, 9 | C 07 D 239/48<br>C 07 D 239/46<br>C 07 D 239/47<br>C 07 D 239/52<br>A 61 K 31/505 |
| A | EP - A1 - 0 343 050 (SYNTHELABO) * Claims 1,2,4; pages 12-14 * -- | 1,7,8, 9 | |
| D,A | EP - A1 - 0 021 121 (WELLCOME) * Claims 1,4,5 * ---- | 1,7,8, 9 | |
| | | | TECHNICAL FIELDS SEARCHED (Int. Cl.5) |
| | | | C 07 D 239/00<br>C 07 D 253/00<br>A 61 K 31/00 |

The present search report has been drawn up for all claims

| Place of search | Date of completion of the search | Examiner |
|---|---|---|
| VIENNA | 03-09-1991 | LUX |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)